# EUROPÄISCHE PATENTSCHRIFT

(11) **EP 2 181 730 B1**
(45) Veröffentlichungstag und Bekanntmachung des Hinweises auf die Patenterteilung: **29.08.2012**
(21) Anmeldenummer: 08168076.1
(22) Anmeldetag: 31.10.2008
(51) Int. Cl.: A61N 7/00, A61B 17/92, A61B 17/00, A61B 17/22

(54) **Instrument zum Erzeugen von stoßwellenartigen Druckwellen zur Behandlung von biologischem Gewebe**
Instrument for producing shock wave-like pressure waves for treating biological tissue
Instrument de production d'ondes de pression de type ondes de choc destiné au traitement de tissus biologiques

(43) Veröffentlichungstag der Anmeldung: 05.05.2010
(73) Patentinhaber: Ferton Holding SA, 2800 Delémont (CH)
(72) Erfinder: Donnet, Marcel, 01630 Saint Jean de Gonville (FR); Benoit, Mathieu, 1805 Jongny (CH); Agbeviade, Kossi, 1054 Morrens (CH)
(74) Vertreter: von Kreisler Selting Werner

(56) Entgegenhaltungen:
- EP-A- 1 574 198
- WO-A-98/57707
- DE-U1-202007 007 921
- US-A- 3 955 563
- US-A- 4 716 890
- US-A- 5 160 336
- US-A- 5 853 384
- US-A1- 2006 069 395

## Beschreibung

Die Erfindung betrifft ein Instrument zur Behandlung von biologischem Gewebe nach dem Oberbegriff des Anspruchs 1 oder 2 .

Derartige Instrumente sind aus der WO9857707 bekannt und dienen dazu, mittels stoßwellenartiger unfokussierter Druckwellen den Heilungsprozess bei Knochenbrüchen, Enthesiopathien, Tendopathien aber auch bei Parodontose zu beschleunigen. Ein weiteres Einsatzgebiet ist die Schmerztherapie im knochennahen Weichteilbereich des Haltungs- und Bewegungsapparates.

Andere zu gleichen therapeutischen Zwecken dienende bekannte Druckimpulsquellen verwenden fokussierte Stosswellen und können nur im eng begrenzten Fokusbereich eine Wirkung erzielen. Für ein befriedigendes Behandlungsergebnis muss allerdings der gesamte Knochenbruchbereich gleichmäßig beschallt werden. Dies verlangt einen aufwändigen Bewegungsmechanismus für die Druckimpulsquelle und ein Ortungssystem, um den Fokus auf den Behandlungsort fokussieren zu können. Die während der Behandlung eingesetzten Ortungssysteme zur Lokalisierung des Behandlungsortes (Ultraschall und Röntgen) können die Schmerzquelle nicht konkret anzeigen und der behandelnde Arzt beschallt mit einer großen Anzahl von Einzelimpulsen den vermuteten Schmerzherd. Eine solche Vorgehensweise ist zudem durch das wiederholte Aufsuchen der Behandlungspositionen sehr zeitintensiv.

Das aus der WO9857707 bekannte gattungsgemäße Instrument zur Behandlung von biologischem Gewebe eines menschlichen oder tierischen Körpers weist einen Druckwellengenerator auf, der unfokussierte Stoßwellen bzw. stoßwellenartige Druckwellen erzeugt, die auf eine einfache und kostengünstige Weise eine gleichmäßige Energieverteilung der Druckwellen auf einen großflächigen Wirkungsbereich ermöglichen. Hierzu ist vorgesehen, dass in einem Gehäuse eine ballistische Einrichtung zum Erzeugen von extrakorporalen stoßwellenartigen Druckwellen und ein im Betrieb auf das biologische Gewebe permanent aufgesetztes Übertragungselement zum Einkoppeln der Druckwellen in den Körper von Lebewesen angeordnet sind. Das Übertragungselement koppelt unfokussierte, ballistisch erzeugte, stoßwellenartige Druckwellen in das biologische Gewebe ein. Die Druckwellen werden von einem auf eine hohe Endgeschwindigkeit, in einem Druckkanal beschleunigten und auf das Übertragungselement auftreffenden hin- und her bewegbaren Schlagteil erzeugt. Das Schlagteil wird von einem über 5 m/s von einem unter Arbeitsdruck stehenden pneumatischen Medium beschleunigt. Der vordere Teil des Druckkanals ist mit einer Gegendruckkammer verbunden, in die das distal vor dem Schlagteil befindliche pneumatische Medium bei der Beschleunigung des Schlagteils auf das Übertragungselement hin einströmen kann.

Die ballistische Einrichtung zum Erzeugen von stoßwellenartigen Druckwellen weist ein in einem Gehäuse geführten, mit Hilfe eines pneumatischen Antriebsmittels hin- und her bewegbares Schlagteil auf, das auf das Übertragungselement einen oder mehrere Kraftstöße ausübt, wobei das Schlagteil infolge der Kraftstöße stoßwellenartige Druckwellen in das nahezu unbewegliche Übertragungselement induziert, die sich bis zur Spitze des Übertragungselementes fortpflanzen. Die Druckwellen mit hohen Druckspitzenwerten werden demzufolge in einfacher Weise ballistisch erzeugt. Die Druckwellen pflanzen sich in dem biologischen Gewebe fort und werden nicht fokussiert. Die mit einem solchen System erzeugten Druckwellen erreichen im Vergleich zu Druckwellengeneratoren mit fokussierter Stoßwelle ähnliche charakteristische Werte in Anstiegszeit, maximaler Druckspitze und Energieflussdichte. Die unfokussierte Druckwelle bereitet sich bis zum Anwendungsort in dem biologischen Gewebe radial aus.

Wesentliche Vorteile des Instrumentes bestehen darin, dass das Instrument einen einfachen, kostengünstigen Aufbau aufweist, dessen Herstellkosten gering sind im Vergleich zu bekannten Druckwellengeneratoren für fokussierte Stoßwellen. Das medizinische Instrument ist als kleines transportables Gerät ausgebildet, das leichter applizierbar ist und ohne Behinderungen auf die zu behandelnde Körperstelle aufgesetzt werden kann. Das Gerät benötigt keine Verbrauchsmaterialien und insbesondere keine Ortungsvorrichtungen, da der Behandlungsbereich in der Nähe der Sondenspitze liegt.

Das bekannte Instrument zielt darauf ab, die stoßwellenartigen Druckwellen nicht zu fokussieren und es dadurch zu ermöglichen, dass diese großflächig eingekoppelt werden können. Ein Ortungssystem ist daher nicht notwendig. Das Instrument eignet sich insbesondere für Behandlungen, bei denen die Sondenspitze auf der Körperoberfläche sehr nah an dem Applikationsort angeordnet werden kann, wie dies beispielsweise bei einem Tennisellenbogen, einem Fersensporn oder auch Erkrankungen der Haut der Fall ist. EP-A-1574198 offenbart ein Instrument mit den Merkmalen der Oberbegriffe der Ansprüche 1 und 2.

Der Erfindung liegt die Aufgabe zugrunde, das eingangs erwähnte Instrument derart weiterzubilden, dass ein Einkopplung einer höheren Druckwellenenergie ohne eine Vergrößerung des Instrumentes und ohne Erhöhung des Arbeitsdrucks ermöglicht wird, und dass eine Erhöhung der Schlagfrequenz ermöglicht wird.

Zur Lösung dieser Aufgabe dienen die Merkmale der Ansprüche 1 und 2.

Die Erfindung sieht in vorteilhafter Weise vor, dass ein schnellschaltendes Ventil das unter dem Arbeitsdruck stehende pneumatische Medium freigibt, wobei eine Steuerschaltung die Öffnungsdauer des Ventils in Abhängigkeit von mindestens einem oder einer Kombination der nachfolgenden Parameter, nämlich den Parametern Arbeitsdruck oder Druck in der Gegendruckkammer, steuert.

Die Erfindung ermöglicht es mit Hilfe einer Steuerschaltung für ein schnell schaltendes Ventil, die einkoppelbare Energie der stoßwellenartigen Druckwellen und damit die Druckspitzenwerte der Druckwellen zu erhöhen, ohne die Abmessungen des Instrumentes zu vergrößern oder den pneumatischen Arbeitsdruck für die ballistische Einrichtung nennenswert erhöhen zu müssen. Ein weiterer Vorteil besteht in der Steigerung der Schlagfrequenz der einzelnen durch das Schlagteil erzeugten Aufschlagimpulse, so dass die Behandlungszeit verkürzt werden kann. Die Verkürzung der Behandlungszeit ist für den Patienten angenehmer. Die Steuerschaltung steuert die Öffnungsdauer des Ventils in Abhängigkeit von dem anliegenden Arbeitsdruck oder dem Druck in der Gegendruckkammer, so dass der Druckaufbau und die Ausnutzung des zur Verfügung stehenden Arbeitsdruckes zum Beschleunigen und zum Rückführen des Schlagteils optimiert werden kann. Die zur Verfügung stehende pneumatische Energie kann um ca. 40% effizienter ausgenutzt werden. Ein weiterer Vorteil einer solchen Steuerung besteht in der Möglichkeit die Stärke des einzelnen Aufschlagimpulses so einzustellen, dass er nur von dem ausgewählten Arbeitsdruck abhängig ist und sich auch bei unterschiedlichen Schlagfrequenzen nicht ändert. Mit anderen Worten, die erfindungsgemäße Steuerung stellt sicher, dass die Schlagintensität bei hohen Schlagfrequenzen konstant ist.

Insbesondere bei hohen Schlagfrequenzen ist die Steuerung der Öffnungsdauer und der Öffnungs- und Schließzeiten vorteilhaft, um den anliegenden Arbeitsdruck effizient zu nutzen und das Schlagteil mit der vorhandenen pneumatischen Energie optimal zu beschleunigen.

Alternativ kann vorgesehen sein, dass ein schnellschaltendes Ventil das unter dem Arbeitsdruck stehende pneumatische Medium in Abhängigkeit von der eingestellten Schlagfrequenz freigibt, wobei eine Öffnung in der Gegendruckkammer den sich aufbauenden Druck in der Gegendruckkammer begrenzt. Die Öffnung weist einen im Vergleich zu dem Volumen der Gegendruckkammer geringen Durchmesser auf, so dass die unter Druck stehende Luft in der Gegendruckkammer nicht sofort vollständig entweichen kann, sondern ein bestimmter Gegendruck für eine bestimmte Zeit aufrechterhalten bleibt.

Die Öffnung in der Gegendruckkammer verbindet die Gegendruckkammer entweder zur Atmosphäre oder zu einer weiteren Kammer, die auch als Druckspeicher ausgebildet sein kann. Besonders bevorzugt ist dabei eine Ausführungsform, in der die Kammer ein Druckspeicher mit elastischen Wänden ist. Die Öffnung in der Gegendruckkammer bildet dabei eine Art Drossel.

Die Öffnung in der Gegendruckkammer zur Atmosphäre hin kann von einer schmalen Blender oder von einem Ventil, insbesondere einem Überdruckventil gebildet sein.

Die Lösung der Aufgabe ist demnach auch erreichbar, wenn der Druck in der Gegendruckkammer während des Druckaufbaus verändert wird. Da der sich in der Gegendruckkammer aufbauende Gegendruck die optimale Beschleunigung des Schlagteils beeinträchtigen kann, ist eine Optimierung auch möglich, wenn die Höhe des Gegendrucks begrenzt oder gesteuert werden kann.

Der Druck in der Gegendruckkammer kann durch ein einstellbares oder steuerbares oder ansteuerbares Überdruckventil gesteuert sein.

Im Falle eines steuerbaren Ventils kann eine Steuerung des Drucks in der Gegendruckkammer in Abhängigkeit von mindestens einem oder einer Kombination der nachfolgenden Parameter erfolgen, nämlich den Parametern Arbeitsdruck, Schlagfrequenz und Druck in der Gegendruckkammer.

Mit Hilfe eines derartigen Überdruckventils kann verhindert werden, dass sich in der Gegendruckkammer ein zu hoher Gegendruck einstellt. Das Überdruckventil kann auch einen bestimmten Grenzwert einstellbar sein, oder variabel in Abhängigkeit von vorgegebenen Parametern steuerbar sein.

Das schnellschaltende Ventil ist vorzugsweise in dem Gehäuse integriert. Die Anordnung des schnellschaltenden Ventils in dem Gehäuse ermöglicht kurze Verbindungswege zu dem Druckkanal bzw. zu der Gegendruckkammer.

Die kurzen Verbindungswege garantieren einen schnellen Druckaufbau insbesondere im Druckkanal, so dass eine energieeffiziente Nutzung des anliegenden Arbeitsdrucks möglich ist.

Bei einem bevorzugten Ausführungsbeispiel gibt das schnellschaltende Ventil das pneumatische Medium aus einem in dem Gehäuse integrierten Zwischenspeicher frei, der das Medium unter einem vorgegebenen Arbeitsdruck zwischenspeichert. Der Zwischenspeicher ist mit dem Versorgungsdruck einer Druckquelle verbunden, wobei der Druck in dem Zwischenspeicher auf einen voreinstellbaren oder steuerbaren Arbeitsdruck eingestellt ist.

Der Zwischenspeicher ist vorzugsweise in der Nähe des Ventils angeordnet.

In gleicher Weise sorgt die Anordnung des Zwischenspeichers in der Nähe des Ventils ebenfalls für kurze Verbindungswege mit wenig Druckverlust, so dass das in dem Zwischenspeicher unter dem Arbeitsdruck befindliche pneumatische Mittel nahezu verlustfrei in den Druckkanal eingeleitet werden kann. Aufgrund der kurzen Verbindungswege kann der Druckaufbau in dem Druckkanal schneller erfolgen.

Der Zwischenspeicher kann in der Nähe des Druckkanals angeordnet sein und pneumatisch über eine im Verhältnis zur Länge des Druckkanals kürzere oder kurze Verbindungsleitung über das Ventil mit dem Druckkanal verbunden sein.

Der Zwischenspeicher kann auch, und zwar alternativ zur Anordnung innerhalb des Gehäuses des Instrumentes in der Zuführungsleitung für das pneumatische Mittel angeordnet sein, die das Gehäuse bzw. das Ventil mit dem pneumatischen Antriebsmittel (Druckquelle) verbindet. Das pneumatische Antriebsmittel besteht dabei vorzugsweise aus einem Kompressor mit einem Druckspeicher.

Das Ventil weist Schaltzeiten von weniger als 5 ms, vorzugsweise weniger als 3 ms, auf. Die kurzen Schaltzeiten des Ventils werden benötigt, um hohe Schlagfrequenzen erzielen zu können. Bevorzugt werden Schaltzeiten von 1 ms und weniger.

Nach einer alternativen Ausführungsform kann für die Rückbewegung des Schlagteils vorgesehen sein, die Gegendruckkammer mit Druck zu beaufschlagen.

Es kann auch vorgesehen sein, dass ein zweites Ventil einen pneumatischen Druck in die Gegendruckkammer für die Rückbewegung des Schlagteils freigibt.

Nach einer weiteren Ausführungsform kann jeweils ein Zwischenspeicher für den Druckkanal und für die Gegendruckkammer vorgesehen sein.

Vorzugsweise ist vorgesehen, dass ein einziges Ventil die Hin- als auch die Rückbewegung des Schlagteils steuert.

Das Volumen des mindestens einen Zwischenspeichers beträgt vorzugsweise mindestens die Hälfte des Volumens des Druckkanalvolumens oder mindestens die Hälfte des bei einem Hub des Schlagteils benötigten Volumens des pneumatischen Mittels. Das Volumen des Zwischenspeichers ist auf diese Weise an das zur Beschleunigung des Schlagteils benötigte Volumen angepasst. Versuche haben gezeigt, dass ein hoher zur Verfügung stehender Arbeitsdruck in der ersten Hälfte der Beschleunigungsphase bereits zu deutlich höheren Endgeschwindigkeiten des Schlagteils geführt hat. Bei einer weiteren Vergrößerung des Zwischenspeichers steigt die Endgeschwindigkeit des Schlagteils nur noch gering an. Wesentlich ist, dass das Druckmedium in dem Zwischenspeicher über einen kurzen Weg mit dem Druckkanal verbunden werden kann, damit der Druckaufbau schnell genug erfolgen kann. Da der Zwischenspeicher mit einem Druckkessel des pneumatischen Antriebsmittels verbunden ist, der unter dem Arbeitsdruck steht, ist gewährleistet, dass der Zwischenspeicher schnell genug wieder aufgefüllt wird, so dass der Arbeitsdruck in dem Druckkanal bis zum Schließsignal für die Steuerung des Ventils aufrechterhalten werden kann.

Bei einem weiteren Ausführungsbeispiel kann ein Wechselventil abwechselnd den Druckkanal oder die Gegendruckkammer mit dem Arbeitsdruck aus dem mindestens einen Zwischenspeicher beaufschlagen.

Ein Wechselventil kann auch abwechselnd den Druckkanal oder die Gegendruckkammer zur Atmosphäre hin öffnen.

In diesem Fall kann der Druckkanal nach Beschleunigung des Schlagteils vom Arbeitsdruck entlastet werden und die Gegendruckkammer nach Rückführung des Schlagteils in seine Ausgangsposition ebenfalls druckentlastet werden.

Bei einem alternativen Ausführungsbeispiel kann ein Wechselventil abwechselnd den Druckkanal oder die Gegendruckkammer mit Druck oder Vakuum beaufschlagen.

Dabei kann ein Druckspeicher sowie ein Vakuumspeicher vorgesehen sein, wobei ein Wechselventil die Speicher abwechselnd mit dem Druckkanal oder mit der Gegendruckkammer verbindet.

Der mindestens eine Druckspeicher aller Ausführungsformen kann elastische Wände aufweisen. In diesem Fall ähnelt der Druckspeicher einer Art Luftballon. Damit wird der Gegendruck in der Gegendruckkammer in geringem Umfang abhängig von der Position des Schlagteils entlang des Druckkanals. In jedem Fall ist der Druckanstieg in der Gegendruckkammer weniger steil, wenn elastische Wände vorgesehen sind. Eine elastische Wand in einem Druckspeicher könnte auch von einem gegen eine Feder arbeitenden Kolben in einer Kolbenzylindereinheit gebildet sein.

Bei einem Verfahren zur Steuerung eines Schlagteils in einem Instrument zur Behandlung von biologischem Gewebe kann entweder das Steuern der Öffnungsdauer eines schnellschaltenden Ventils in Abhängigkeit von mindestens einem oder einer Kombination der nachfolgenden Parameter, nämlich dem Arbeitsdruck oder des Drucks in der Gegendruckkammer vorgesehen sein oder das Begrenzen des sich aufbauenden Drucks in der Gegendruckkammer. Dies kann auch in Abhängigkeit von mindestens einem oder einer Kombination der nachfolgenden Parameter, nämlich den Parametern Arbeitsdruck, Schlagfrequenz und Druck in der Gegendruckkammer erfolgen. Auch im Fall der zuletzt genannten Alternative kann das Steuern des schnellschaltenden Ventils hinsichtlich Öffnungszeiten und/oder Öffnungsdauer vorgesehen sein.

Insbesondere bei orthopädischen Anwendungen ist es vorteilhaft, eine Vielzahl von Druckwellen in das biologische Gewebe einzukoppeln, um eine optimale Wirkung zu erzielen. Die ballistische Einrichtung ist daher vorzugsweise so ausgestaltet, dass eine periodische Hin- und Herbewegung des Schlagteils möglich ist. Die Schlagfrequenz beträgt bis zu 50 Hz, vorzugsweise bis zu 60 Hz.

Zwischen der Sondenspitze und der Einkoppelstelle auf dem biologischen Gewebe kann ein Impedanzanpassungsmedium angeordnet sein, das die Einkopplung der Druckwelle in das biologische Gewebe verbessert. Ein geeignetes pastenförmiges Impedanzanpassungsmedium ist beispielsweise ein Ultraschallgel oder eine andere pastöse Masse, z. B. Vaseline.

Die Länge des Übertragungselementes kann im Bereich zwischen ca. 20 und 100 mm liegen. Über unterschiedliche und auswechselbare Übertragungselemente kann eine Anpassung an die gewünschte Behandlung erfolgen.

Im folgenden werden unter Bezugnahme auf die Zeichnungen Ausführungsbeispiele der Erfindung näher erläutert.

Es zeigen :
- Fig. 1: eine Darstellung des Instrumentes im Querschnitt,
- Fig. 2: das Instrument gemäß Fig. 1 in einer anderen Schaltstellung des Ven- tils, und
- Fig. 3: ein zweites Ausführungsbeispiel des Instrumentes.

Das medizinische Instrument ermöglicht das Behandeln von biologischem Hart- und Weichgewebe, insbesondere zur Heilung von Knochenleiden, wie Knochenbrüchen, Enthesiopathien, Tendopathien und Parodontose, sowie eine Schmerztherapie im knochennahen Weichteilbereich des Haltungs-und Bewegungsapparates, indem ballistisch erzeugte Druckwellen unfokussiert über ein an der Kontaktfläche mit dem Körper stumpfes Übertragungselement 2 in das biologische Gewebe eingekoppelt werden.

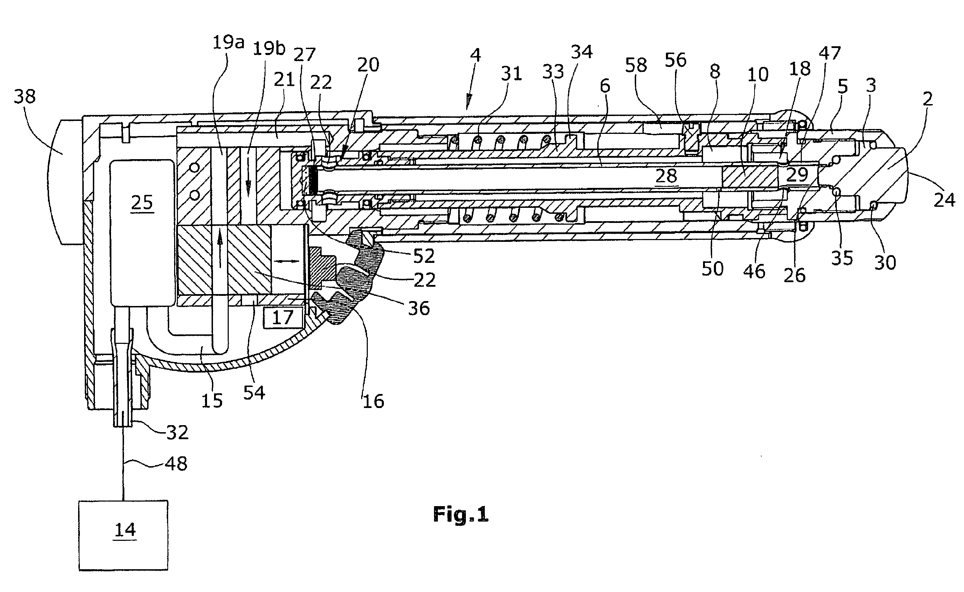

Das in Fig. 1 gezeigte Handstück 1 besteht aus einem Gehäuse 4, das einen Druckkanal 6 aufnimmt, in dem ein Schlagteil 10 mit Hilfe pneumatischer Antriebsmittel 14 in Verbindung mit einer Gegendruckkammer 8, die den Druckkanal 6 beispielsweise koaxial ringförmig umgibt, zwischen zwei Endpositionen hin- und herbewegt wird. Der Beschleunigungsweg weist bei einem pneumatischen Antrieb ca. 100 bis 250 mm Länge auf.

In der proximalen Endposition des Schlagteils 10 kann am proximalen Ende 20 des Druckkanals 6 ein Magnethalter und/oder ein beispielsweise elastisches Anschlagelement 27 angeordnet sein. Ein Magnethalter kann das metallische Schlagteil 10 in seiner proximalen Endposition festhalten, bis erneut ein Ventil 16 einen über den Anschluss 32 aufgebrachten pneumatischen Druck freigibt und das Schlagteil 10 in Richtung auf das distale Ende 18 des Druckkanals 6 beschleunigt. Die sich in Bewegungsrichtung des Schlagteils 10 vor dem Schlagteil 10 befindliche Luft wird über an dem distalen Ende 18 des Druckkanals 6 befindliche Öffnungen 46 in die Gegendruckkammer 8 geleitet.

Durch die Beschleunigung des Schlagteils 10 trifft dieses mit einer hohen ballistischen Endgeschwindigkeit von beispielsweise über 5 m/s auf eine distal vor dem Druckkanal 6 angeordnete Endfläche 26 des Übertragungselementes 2. Das Übertragungselement 2 weist eine plane oder gekrümmt geformte Kontaktfläche 24 auf. Das Schlagteil 10 übt einen oder mehrere Kraftstöße auf das Übertragungselement 2 aus, das die von dem Schlagteil 10 in das Übertragungselement 2 induzierten Druckwellen an die Kontaktfläche 24 weiterleitet und dort in ein biologisches Gewebe einkoppelt.

Das Übertragungselement 2 ist in dem Gehäuse 4 linear und vorzugsweise koaxial zu dem Schlagteil 10 geführt. Das Gehäuse 4 weist ein Kopfteil 5 auf, das für ein Auswechseln des Übertragungselementes 2 abschraubbar ist. Das Übertragungselement 2 ist in einer Bohrung des Kopfteils 5 gelagert und kann mit Hilfe eines nicht dargestellten O-Rings im vorderen Abschnitt des Kopfteils 5 abgedichtet sein. Ein Ringbund 3 des Übertragungselementes 2 dient als Anschlagelement wobei zwischen dem Ringbund 3 des Übertragungselementes 2 und dem Kopfteil 5 des Gehäuses 4 ein Feder/Dämpfungselement 30 angeordnet ist, das das Übertragungselement 2 von dem Gehäuse 4 in Axialrichtung entkoppelt. Für die Einkopplung der Druckwelle in das biologische Gewebe ist eine Verlagerung des Übertragungselementes nicht notwendig und weitgehend unerwünscht, um Verletzungen zu vermeiden.

Der sich in der Gegendruckkammer 80 aufbauende Gegendruck genügt bei Wegfall des an dem pneumatischen Anschluss 32 anliegenden Druckes, um das Schlagteil 10 von der distalen Endlage an dem Übertragungselement 2 in die proximale Endlage zurückzubewegen. Der pneumatisch regelbare Druck an dem Anschluss 32 kann beispielsweise bis zu 6 bar, vorzugsweise 4 bar, betragen. Das Schlagteil 10 kann zwecks Anpassung an bestimmte Längen des Übertragungselementes 2 oder zum Erzeugen einer bestimmten Charakteristik der Druckwelle hinsichtlich Länge, Masse und maximale Aufschlaggeschwindigkeit unterschiedlich gewählt werden und durch Abschrauben des Kopfteils 5 leicht ausgetauscht werden. Typische maximale Druckwerte an der Spitze des Übertragungselementes 2 liegen zwischen 2 und 25 MPa bei Anstiegszeiten von 0,5 bis 3 µs und Energieflussdichten zwischen 0,05 und 0,6 mJ/mm².

Die proximale Endfläche 26 des Übertragungselementes 2 kann in etwa den gleichen Durchmesser wie das Schlagteil 10 aufweisen. Die Länge des Schlagteils 10 ist vorzugsweise größer als sein Durchmesser. Damit werden bessere Führungseigenschaften in dem Druckkanal 6 erreicht. Außerdem kann mit Hilfe einer unterschiedlichen Länge des Schlagteils 10 die Masse in einfacher Weise variiert werden, ohne dass der Durchmesser des Druckkanals 6 und der Eintrittsgrenzfläche 26 des Übertragungselementes 2 verändert werden müssen.

Zur Erzeugung eines pneumatischen Beschleunigungsimpulses sind schnellöffnende und -schließende Ventile geeignet, die in der zuführenden Leitung angeordnet sind. Da für den Beschleunigungsvorgang kurzzeitig größere Luftmengen benötigt werden, ist es vorteilhaft, in der zuführenden Leitung 48 einen Zwischenspeicher 25 vorzusehen, der in idealer Weise innerhalb des Gehäuses 4 und nahe dem Ventil 16 angeordnet ist.

Zum schnelleren Öffnen oder Schalten des Ventils 16 wird eine elektrische oder elektronische Steuerschaltung 17 verwendet. Bei einer derartigen Steuerschaltung 17 können besonders hohe Ströme in der Öffnungsphase bereitgestellt werden, die später auf ein Normalmaß angesenkt werden. Mit derartigen elektrisch ansteuerbaren Ventilen 16 lassen sich Schaltzeiten von unter 1 ms erreichen. Generell sollten die Schaltzeiten des Ventils 16 unter 5 ms, vorzugsweise unter 3 ms, liegen. Die Öffnungsdauer des Ventils beträgt je nach Arbeitsdruck und Schlagfrequenz, beispielsweise zwischen 3 und 35 ms.

Zur Verringerung der benötigten Mengen an pneumatischen Medium, vorzugsweise Luft, ist es von Vorteil, das Volumen welches für den einzelnen Druckimpuls benötigt wird, so klein wie möglich zu halten.

Ein elektrischer Druckschalter 22 ist mit der Steuerschaltung 17 verbunden, die das elektrisch bzw. elektronisch ansteuerbare Ventil 16 steuert. Insofern ist das Ventil 16 in das Gehäuse 4 des Instrumentes integriert.

Mit dem Instrument zur Behandlung von biologischem Gewebe soll nicht nur einzelne Druckimpulse erzeugt werden, vielmehr werden für medizinische Indikationen eine größere Anzahl von Druckimpulsen benötigt. Typische Werte liegen im Bereich zwischen 1000 und 5000 Impulsen, in bestimmten Einzelfällen auch deutlich höher. Daher soll das Instrument in der Lage sein, diese Anzahl an Impulsen in einer kurzen Zeit bereitzustellen, um die Behandlungsdauer nicht unnötig in die Länge zu ziehen.

Das Volumen der Gegendruckkammer 8 ist dabei so einzustellen, dass ein ausreichend hoher Gegendruck aufgebaut wird, um Schlagteil 10 zurückzuführen. Je höher der Gegendruck ist, umso schneller wird das Schlagteil 10 zurückgeführt. Ein zu hoher Gegendruck bremst aber das Schlagteil 10 in seiner Vorwärtsbewegung stark ab und verringert damit seine Aufschlagwirkung auf das Übertragungselement 2.

Es hat sich außerdem gezeigt, dass nicht nur das vor dem Schlagteil 10 liegende Luftvolumen den Gegendruck in der Gegendruckkammer 8 aufbaut, sondern auch ein Leckvolumen aufgrund des Arbeitsdrucks hinter dem Schlagteil 10 zusätzlich an dem Schlagteil 10 vorbei in die Gegendruckkammer 8 gelangt und den dortigen mittleren Druck beeinflusst. Dadurch werden die Parameter zur Rückführung des Schlagteils 10 verändert und einhergehend damit ändern sich die voreingestellten Leistungswerte des Gerätes mit zunehmender Behandlungsdauer. Um dem entgegenzuwirken, kann durch zusätzliche Mittel der Druck in der Gegendruckkammer 8 eingestellt werden.

Der Druck in der Gegendruckkammer 8 kann beispielsweise dadurch begrenzt werden, dass die Gegendruckkammer 8 über eine drosselartige Öffnung 61 in der Gegendruckkammer 8 und über kleine Öffnungen 62 mit der Atmosphäre verbunden wird.

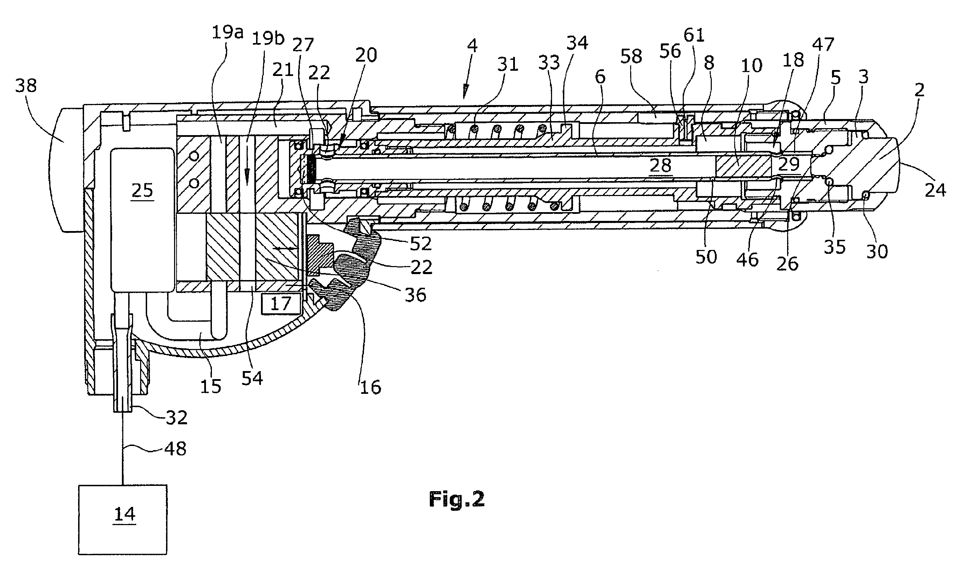

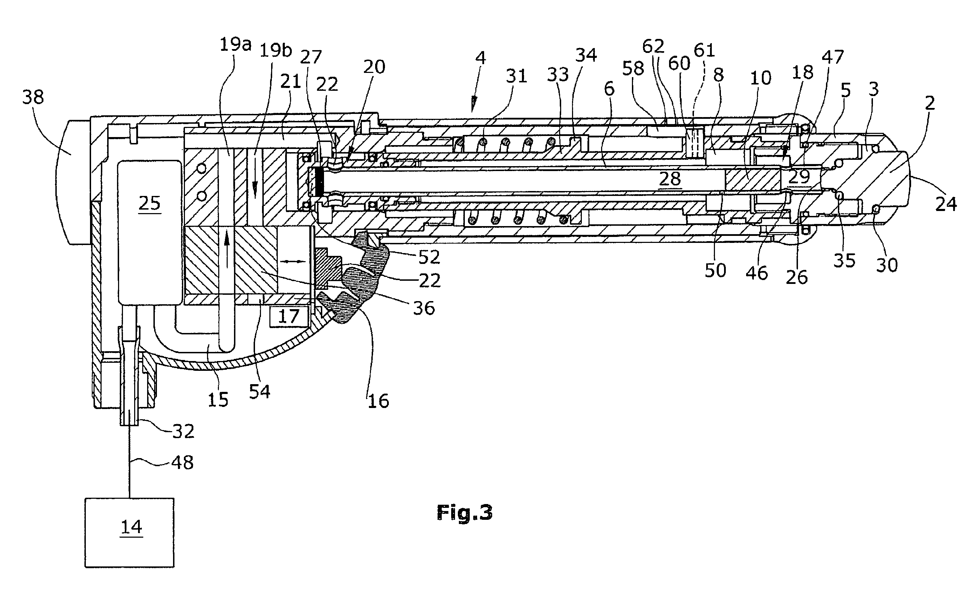

Die Öffnung 61 in der Gegendruckkammer 8 kann beispielsweise in der in Fig. 2 ersichtlichen Schraube 56 angeordnet sein oder in dem Ventil 60, wie in Fig. 3 gezeigt.

Durch diese Öffnungen 61,62 zur Atmosphäre kann das pneumatische Medium ausströmen und damit den Gegendruck in der Gegendruckkammer 8 verringern. Ist der Druck in der Gegendruckkammer 8 besonders hoch, so strömt damit auch eine größere Luftmenge durch die Öffnungen 61,62.

Alternativ kann auch ein Ventil 60 vorgesehen sein, das eine Öffnung 61 zur Atmosphäre öffnet oder schließt. Dabei kann es sich um Druckbegrenzungsventil handeln oder ein ansteuerbares Ventil, das in Kombination mit einer Druckmessvorrichtung in Abhängigkeit von einem voreingestellten Gegendruck in der Gegendruckkammer 8 öffnet oder schließt.

Alternativ kann ein Überdruckventil vorgesehen sein, das selbsttätig bei Überschreiten eines einzustellenden Wertes öffnet.

Wird in der Gegendruckkammer 8 ein höherer Druck benötigt, so kann die Gegendruckkammer 8 auch über ein schaltbares Ventil 60 mit einem Druckspeicher verbunden sein, wobei eine Verbindung mit dem Zwischenspeicher 25 erfolgen kann oder mit einem separaten Druckspeicher für die Gegendruckkammer 8.

Die in der Nähe des distalen Endes des Gehäuses 4 in die zylindrische Hülse 33 eingeschraubte Schraube 56 dient als Verdrehsicherung und Anschlagmittel zur Begrenzung des Federweges der Feder 31. Die Schraube 56 ist in einer Nut 58 des Gehäuses 4 geführt. In der Schraube kann auch die Öffnung 61 vorgesehen sein.

Anstelle der Schraube 56 kann auch ein Ventil 60 mit einer Öffnung 61 an dieser Stelle angeordnet sein, das die Gegendruckkammer 8 mit der Atmosphäre verbindet. Wie aus Fig. 3 ersichtlich, kann die Gegendruckkammer 8 über das Ventil 60, z.B. ein Druckbegrenzungsventil, mit der Atmosphäre verbunden sein, so dass der maximale Druck in der Gegendruckkammer 8 gesteuert werden kann. Für diesen Fall hat die Außenwand des Gehäuses 4 im Bereich der Nut 58 Öffnungen 62 zur Atmosphäre hin.

Eine Verbindung der Gegendruckkammer 8 zur Atmosphäre hin über eine Öffnung 61 oder ein Ventil 60 zur Begrenzung des sich in der Gegendruckkammer 8 aufbauenden Drucks ist sowohl, in dem Fall anwendbar, in dem das schnellschaltende Ventil hinsichtlich der Öffnungsdauer gesteuert wird, aber auch in dem Fall, in dem das Ventil nur aufgrund der Schlagfrequenz gesteuert wird und die Ventilöffnungsdauer konstant ist.

Die Begrenzung oder Steuerung des Drucks in der Gegendruckkammer 8 kann aber auch in Kombination mit einem schnellschaltenden Ventil 16 mit variablen Steuerzeiten und variabler Öffnungsdauer des Ventils erfolgen.

In der einfachsten Ausführungsform weist die Gegendruckkammer 8 lediglich eine schmale Öffnung 61 in der Art einer Drosselstelle auf, die die Gegendruckkammer 8 entweder mit der Atmosphäre oder mit einer weiteren Kammer z.B. die unterhalb der Nut 58 angeordnete Ringkammer, in der die Hülse 33 angeordnet ist, verbindet. Die Öffnung in der Art einer Drosselstelle ermöglicht, dass der Gegendruck in der Gegendruckkammer sich insbesondere bei hohem Drücken reduzieren kann.

Führt die Öffnung zu einer weiteren Kammer, so ist diese vorzugsweise als elastischer Druckspeicher ausgeführt, z.B. in der Art eines Luftballons oder in der Art einer Kolbenzylindereinheit, bei der Kolben gegen die Kraft einer Feder verlagert werden kann.

Um die Bewegung des Schlagteils 10 zu kontrollieren, können auch die Steuerzeiten für das Ventil 16 und die Öffnungsdauer des Ventils 16 gesteuert werden. Das Schlagteil 10 wird nicht nur durch den Gegendruck in der Gegendruckkammer 8 in seine Ausgangsposition zurückgeführt, sondern der Aufprall auf das Übertragungselement 2 erteilt dem Schlagteil 10 auch eine kinetische Energie, die das Schlagteil 10 wieder in Richtung zur proximalen Ausgangsposition zurückstößt. Bei entsprechender Gestaltung der Begrenzungswand 27 am proximalen Ende 20 des Druckkanals 6 kann auch durch die hintere Begrenzungswand 27 ein elastischer Stoß bei Kollision mit dem elastischer Stoß bei Kollision mit dem Schlagteil 10 stattfinden, so dass durch den Rückprall ein Teil der kinetischen Energie auch für die Vorwärtsbewegung des Schlagteils 10 genutzt werden kann. Um dies zu erreichen, ist es zweckmäßig, auf die Schlagteilbewegung aktiv Einfluss zu nehmen. Dies kann beispielsweise dadurch geschehen, dass die Steuerzeiten für das Ventil 16 variabel eingestellt werden.

Die Steuerschaltung 17 kann die Öffnungsdauer des Ventils 16 in Abhängigkeit von mindestens einem oder einer Kombination der nachfolgenden Parameter, nämlich den Parametern Arbeitsdruck oder Druck in der Gegendruckkammer 8 steuern. Außerdem können Öffnungs- und Schließzeitpunkt des Ventils 16 gesteuert werden. Alternativ oder zusätzlich kann die Steuerschaltung 17 den maximalen Druck in der Gegendruckkammer 8 in Abhängigkeit von mindestens einem oder einer Kombination der nachfolgenden Parameter, nämlich den Parametern Arbeitsdruck, Schlagfrequenz und den Druck in der Gegendruckkammer 8 steuern. Mit Hilfe der Steuerung des Öffnungszeitpunktes und der Öffnungsdauer des Ventils 16 lässt sich das Schlagteil 10 in seiner Vor- und Zurückbewegung derart optimieren, dass die Schlagintensität bei vergleichbarem zur Verfügung stehenden Arbeitsdruck um bis zu 40% erhöht wird, wobei außerdem die Schlagfrequenz erhöht werden kann. Dabei wird die Verbesserung der Schlagintensität und der Schlagfrequenz ohne Vergrößerung des Instrumentes und ohne Erhöhung, jedenfalls ohne nennenswerte Erhöhung des Arbeitsdruckes erreicht.

Zur besseren Kontrolle und Steuerung der Schaltpunkte des Ventils 16 können auch Messmittel vorgesehen sein, die die Position oder die Bewegung des Schlagteils 10 ermitteln und damit weitere Informationen zur Steuerung der Schaltzeiten an die Steuerschaltung 17 liefern. Beispielsweise kann der Druck in der Gegendruckkammer 8 gemessen werden, sowie das Erreichen einer bestimmten Position des Schlagteils 10 im Druckkanal, beispielsweise durch optische Messmethoden.

Dies bedeutet, dass das Detektionssignal, das anzeigt, dass das Schlagteil 10 eine vorgegebene Position in dem Druckkanal 6 erreicht hat, zur Steuerung des Ventils 16 verwendet werden kann. So kann beispielsweise die Druckbeaufschlagung des Druckkanals 6 bereits enden, bevor das Schlagteil 10 auf das Übertragungselement 2 aufschlägt.

Auch kann der Gegendruck in der Gegendruckkammer 8 auf einen Wert begrenzt werden, der ein genügend schnelles Zurückführen des Schlagteils 10 in die proximale Endposition gewährleistet.

Für ein wiederholtes Aufschlagen ist es zweckmäßig, wenn das Schlagteil 10 selbsttätig rückstellbar ist. Für die Rückführung des Schlagteils 10 in seine Initialposition sind mehrere Lösungen möglich. So kann auch die Rückführung des Schlagteils 10, mit Hilfe eines gesteuerten pneumatischen Pulses erfolgen. Ein zweites Ventil kann diese Aufgabe übernehmen. Zur Kosteneinsparung könnte auch nur ein einziges Ventil 16 verwendet werden, das in der Lage ist, abwechselnd einen Druckluftpuls für die Beschleunigung und die Rückführung bereit zu stellen.

In der Praxis bewährt hat sich eine Art pneumatische Feder. Dabei bildet der Luftraum vor dem Schlagteil und eine damit verbundene Gegendruckkammer 8 einen abgeschlossenen Bereich, der durch die Schlagteilbewegung in Vorwärtsrichtung verkleinert wird. In dieser Gegendruckammer 8 erhöht sich dadurch der Druck und so kann das Schlagteil 10 nach dem Abschalten des beschleunigenden Druckluftpulses wieder in seine Ausgangsposition zurückgeführt werden. Das Volumen dieser Gegendruckkammer 8 ist so auszuwählen, dass einerseits die Vorwärtsbewegung des Schlagteils 10 durch den sich aufbauenden Gegendruck nicht zu stark behindert wird, andererseits der aufgebaute Gegendruck ausreichend hoch ist, um das Schlagteil 10 schnell genug in seine proximale Ausgangsposition zurückzubringen.

Befindet sich das Schlagteil 10 bewegungslos in der Nähe des Übertragungselementes 2, entfernt von seiner Ausgangsposition, so kann es ohne Hilfsmittel nicht in einen funktionstüchtigen Zustand am proximalen Ende 20 versetzt werden. Ein Druckluftpuls hätte keine Wirkung. Um das Gerät auch aus solchen Situationen in einen funktionstüchtigen Zustand zu bringen, kann es auch eine kleine Verbindungsöffnung zwischen der Gegendruckkammer 8 und dem hinteren Teil des Druckkanals 6 vorgesehen sein. Dies bewirkt, dass in die Gegendruckkammer 8 Luft einströmen kann und so der Druck darin ansteigt. Damit kann das Schlagteil 10 dann wieder in seine ursprüngliche proximale Ausgangsposition zurückgeführt werden.

Das in Fig. 1 dargestellte Instrument wird über die Leitung 48 mit einem pneumatischen Antriebsmittel 14, vorzugsweise einem Kompressor mit Druckkessel unter einem Versorgungsdruck über den Anschluss 32 vorsorgt. Der Versorgungsdruck wird verwendet, um einen in dem Gehäuse 4 befindlichen Zwischenspeicher 25 auf einen voreinstellbaren Arbeitsdruck, beispielsweise 3 oder 4 bar zu bringen.

Zu bevorzugen ist allerdings, dass der Versorgungsdruck des Druckkessels des pneumatischen Antriebsmittels 14 bereits den Arbeitsdruck aufweist, so dass der Zwischenspeicher 25 in dem Gehäuse 4 stets mit unter dem gewählten Arbeitsdruck stehenden pneumatischen Medium noch während einer Entleerung des Zwischenspeichers 25 schnell aufgefüllt werden kann.

Das pneumatische Medium, vorzugsweise Luft, wird über die Leitung 15 und ein schnellschaltendes, vorzugsweise elektromagnetisch gesteuertes Ventil 16 entweder über die Leitungen 19a und 21, sowie die auf dem Umfang des Druckkanals 6 gleichmäßig verteilt angeordneten Öffnungen 22 dem Druckkanal 6 zugeführt, wodurch das Schlagteil 10 von einer Ausgangsposition am proximalen Ende 20 des Druckkanals 6 in eine distale Position beschleunigt werden kann. Das Schlagteil 10 schlägt am distalen Ende 18 des Druckkanals 6 geschossartig mit hoher Geschwindigkeit auf die hintere Endfläche 26 des Übertragungselementes 2 aufschlägt. Da das Übertragungselement 2 sich im wesentlichen in Ruhe befindet, wird die Schlagenergie in eine stoßwellenartige Druckwelle umgewandelt, die an der distalen Frontfläche 24 des Übertragungselementes 2 in den Körper eingekoppelt wird. Die sich aufgrund des Arbeitsdruckes in dem Raum 28 hinter dem Schlagteil 10 ausdehnende Luft führt in dem Raum 29 vor dem Schlagteil 10 zu einer Verdichtung der Luft, die über auf dem Umfang des Druckkanals 6 verteilt angeordnete Öffnungen 46 in eine vorzugsweise den Druckkanal 6 ringzylindrisch umgebende Gegendruckkammer 8 geleitet wird. Das distale Ende des Druckkanals 6 kann in dem Bereich distal vor den Öffnungen 46 Schlitze 47 aufweisen, damit die vor dem Schlagteil 10 befindliche Luft dennoch über die Öffnungen 46 in die Gegendruckkammer 8 entweichen kann. In der Nähe des distalen Endes 18 kann, wie aus den einzigen Figuren ersichtlich, hinter dem Schlagteil 10 in der gezeigten Position eine Verbindungsöffnung 50 zwischen dem Druckkanal 6 und der Gegendruckkammer 8 bestehen, die dazu dient, den in dem Raum 28 vorhandenen Druck auch in die Gegendruckkammer 8 zu leiten, sobald das Schlagteil 10 die Verbindungsöffnung 50 passiert hat. Zwischen den Kammern kann daher ein gewisser Druckausgleich stattfinden, wobei die Schnelligkeit des Druckausgleichs durch den Druckmesser der Verbindungsöffnung 50 gesteuert werden kann.

Wesentlich ist, dass nach dem Aufprall des Schlagteils 10 auf das Übertragungselement 2 ein ausreichender Druck in der Gegendruckkammer 8 vorhanden ist, der die Rückführung des Schlagteils 10 in seine proximale Position ermöglicht. Hierzu ist auch von Bedeutung, dass ein Teil der kinetischen Energie des Schlagteils 10 nach dem Aufprall auf das Übertragungselement 2 durch den elastischen Rückstoß von dem Übertragungselement 2 erhalten bleibt, so dass sich der in der Gegendruckkammer 8 befindliche Druck in der Kammer 29 entfalten kann, um das Schlagteil 10 zurückzuführen.

Auch an dem proximalen Ende 20 des Druckkanals 6 ist Vorsorge getroffen, dass der Arbeitsdruck bei dem erstmaligen Auslösen einer Schlagimpulsfolge hinter das Schlagteil 10 gelangen kann, um das Schlagteil 10 zu beschleunigen. Bei Ausführung von mehreren Impulsfolgen kann die hintere Begrenzungswand 27 des Druckkanals 6 elastische Eigenschaften aufweisen, so dass das Schlagteil 10 in der Art eines elastischen Stoßes von der Begrenzungswand 27 zurückprallen kann. Hinter der Begrenzungswand 27 kann sich ein Magnet 52 befinden, der das Schlagteil 10 in der proximalen Endposition halten kann, wenn die Impulsfolgen beendet werden.

Das Übertragungselement mit dem Kopfteil 5, dem Druckkanal 6 und einer die Gegendruckkammer 8 nach außen begrenzenden Hülse 33 kann gegen die Kraft einer Druckfeder 31 in proximaler Richtung bewegt werden, damit das Übertragungselement 2 stets mit einem definierten maximalen Anpressdruck gegen das biologische Gewebe angedrückt werden kann. Die Druckfeder 31 liegt einerseits an einem Bund 34 der Hülse 33 an und andererseits am Gehäuse 4. Die Druckfeder 31 verhindert somit, dass der von einer Bedienungsperson beispielsweise über die Endkappe 38 ausgeübte Anpressdruck des Übertragungselementes 2 auf das biologische Gewebe zu hoch wird.

Bei Betätigen des Schalters 22 wird mit Hilfe der elektronischen Steuerschaltung 17 das Ventil 16 in die in Fig. 1 dargestellten Position verlagert, so dass der Arbeitsdruck aus dem Zwischenspeicher 25 in den Druckkanal 6 in den Raum 28 eintreten kann. Die Steuerschaltung 17 kann dann in Abhängigkeit von dem anliegenden Arbeitsdruck oder dem Druck in der Gegendruckkammer 8 die Öffnungsdauer des Ventils 16 steuern, und insbesondere auch die Öffnungs- und Schließzeiten im Falle von Impulsfolgen festlegen. Auf diese Weise kann die zur Verfügung stehende pneumatische Energie effizienter genutzt werden. Die Steuerschaltung 17 kann auch in Abhängigkeit der obigen Parameter Zeitverzögerungen durch Trägheitseffekte berücksichtigen, so dass die Schaltzeitpunkte im Sinne einer effizienteren Energienutzung jeweils optimal gesteuert werden.

In der Schließstellung des Ventils 16, wie aus Fig. 2 ersichtlich, ist einerseits die Verbindung zwischen der Verbindungsleitung 15 und dem Kanal 19a verschlossen, als auch die Verbindung des Kanals 19b mit dem Ausgang 54 des Ventils 16 verbunden, der zur Druckentlastung des Druckkanals 6 mit der Atmosphäre verbunden sein kann.

## Patentansprüche

1. Instrument zur Behandlung von biologischem Gewebe, mit einem Gehäuse (4), in dem eine ballistische Einrichtung zum Erzeugen von extrakorporalen stoßwelfenartigen Druckwellen und ein auf das biologische Gewebe permanent aufgesetztes Übertragungselement (2) zum Einkoppeln der Druckwellen in den Körper von Lebewesen angeordnet sind, wobei das Übertragungselement (2) unfokussierte, ballistisch erzeugte, stoßwellenartige Druckwellen in das biologische Gewebe einkoppelt, die von einem auf eine hohe Endgeschwindigkeit von über 5 m/s von einem unter Arbeitsdruck stehenden pneumatischen Medium in einem Druckkanal (6) beschleunigten und auf das Übertragungselement (2) auftreffenden hin- und her bewegbaren Schlagteil (10) erzeugbar ist, wobei der vordere Teil des Druckkanals (6) mit einer Gegendruckkammer (8) verbunden ist, in die das distal vor dem Schlagteil (10) befindliche pneumatische Medium bei der Beschleunigung des Schlagteils (10) auf das Übertragungselement (2) hin einströmen kann, wobei
ein schnellschaltendes Ventil (16) das unter dem Arbeitsdruck stehende pneumatische Medium in den Druckkanal (6) freigibt, **dadurch gekennzeichnet, dass** eine Steuerschaltung (17) die Öffnungsdauer des Ventils (16) in Abhängigkeit von mindestens einem oder einer Kombination der nachfolgenden Parameter, nämlich den Parametern Arbeitsdruckoder Druck in der Gegendruckkammer (8), steuert.

2. Instrument zur! Behandlung von biologischem Gewebe, mit einem Gehäuse (4), in dem eine ballistische Einrichtung zum Erzeugen von extrakorporalen stoβwellenartigen Druckwellen und ein auf das biologische Gewebe permanent aufgesetztes Übertragungselement (2) zum Einkoppeln der Druckwellen in den Körper von Lebewesen angeordnet sind, wobei das Übertragungselement (2) unfokussierte, ballistisch erzeugte, stoßwellenartige Druckwellen in das biologische Gewebe einkoppelt, die von einem auf eine hohe Endgeschwindigkeit von über 5 m/s von einem unter Arbeitsdruck stehenden pneumatischen Medium in einem Druckkanal (6) beschleunigten und auf das Übertragungselement (2) auftreffenden hin- und her bewegbaren Schlagteil (10) erzeugbar ist, wobei der vordere Teil des Druckkanals (6) mit einer Gegendruckkammer (8) verbunden ist, in die das distal vor dem Schlagteil (10) befindliche pneumatische Medium bei der Beschleunigung des Schlagteils (10) auf das Übertragungselement (2) hin einströmen kann, wobei
ein schnellschaltendes Ventil (16) das unter dem Arbeitsdruck stehende pneumatische Medium in den Druckkanal (6) in Abhängigkeit von der eingestellten Schlagfrequenz freigibt, **dadurch gekennzeichnet, dass** eine Öffnung (61) in der Gegendruckkammer (8) den sich aufbauenden Druck in der Gegendruckkammer (8) begrenzt.

3. Instrument nach Anspruch 2, **dadurch gekennzeichnet, dass** die Öffnung (61) der Gegeridruckkammer (8) hin von einer schmalen Blende oder von einem Überdruckventil (60) gebildet ist.

4. Instrument nach einem der Ansprüche 1 bis 3, **dadurch gekennzeichnet, dass** der Druck in der Gegendruckkammer (8) durch ein steuerbares Überdruckventil (60) in Abhängigkeit von mindestens einem oder einer Kombination der nachfolgenden Parameter, nämlich den Parametern Arbeitsdruck, Schlagfrequenz und/oder Druck in der Gegendruckkammer (8) gesteuert ist.

5. Instrument nach Anspruch 1 bis 4, **dadurch gekennzeichnet, dass** das schnellschaltende Ventil (16) das pneumatische Medium aus einem in das Gehäuse (4) integrierten Zwischenspeicher (25) freigibt, der das Medium unter einem vorgegebenen Arbeitsdruck zwischenspelchert.

6. Instrument nach einem der Ansprüche 1 bis 5, **dadurch gekennzeichnet, dass** der Zwischenspeicher (25) in der Nähe des Druckkanals (6) angeordnet ist und! pneumatisch über eine im Verhältnis zur Länge des Druckkanals (6) kürzere oder kurze Verbindungsleitung (19a, 19b) über das Ventil (16) mit dem Druckkanal (6) verbunden ist.

7. Instrument nach einem der Ansprüche 1 bis 6, **dadurch gekennzeichnet, dass** der Zwischenspeicher (25) in der Zuführungsleitung (48) für das pneumatische Mittel angeordnet ist.

8. Instrument nach einem der Ansprüche 1 bis 7, **dadurch gekennzeichnet, dass** ein zweites Ventil einen pneumatischen Druck in die Gegendruckkammer (8) für die Rückbewegung des Schlagteils (10) freigibt.

9. Instrument nach einem der Ansprüche 1 bis 8, **dadurch gekennzeichnet, dass** ein einziges Ventil (16) die Hin- als auch die Rückbewegung des Schlagteils (10) steuert.

10. Instrument nach einem der Ansprüche 1 bis 9, **dadurch gekennzeichnet, dass** das Volumen des mindestens einen Zwischenspeichers (25) mindestens die Hälfte des Volumens des Druckkanalvolumens oder mindestens die Hälfte des bei einem Hub des Schlagteils (10) benötigten Volumens des pneumatischen Mittels beträgt.

11. Instrument nach einem der Ansprüche 1 bis 10, **dadurch gekennzeichnet, dass** das Ventil (16) abwechseln den Druckkanal (6) oder die Gegendruckkammer (8) mit dem Arbeitsdruck aus dem mindestens einen Zwischenspeicher (25) beaufschlagt.

12. Instrument nach einem der Ansprüche 1 bis 11, **dadurch gekennzeichnet, dass** das ventil (16) abwechselnd den Druckkanal (6) oder die Gegendruckkammer (8) zur Atmosphäre hin öffnet.

13. Instrument nach einem der Ansprüche 5 bis 12, **dadurch gekennzeichnet, dass** der Zwischenspeicher (25) elastische Wände oder mindestens eine elastische Wand aufweisen.

## Claims

1. An instrument for the treatment of biological tissue, comprising a housing (4) in which are arranged a ballistic means for generating extracorporeal shock wave-like pressure waves and a transfer element (2) permanently placed on the biological tissue for coupling said pressure waves into the bodies of living beings, said transfer element (2) coupling non-focused, ballistically generated, shock wave-like pressure waves into said biological tissue, which are generated by a reciprocating striking member (10) accelerated to a final velocity of more than 5 m/s by means of a pneumatic medium under working pressure in a pressure channel (6) and striking said transfer element (2), the front part of said pressure channel (6) being connected with a back-pressure chamber (8) into which the pneumatic medium present distally of the striking member (10) can flow as the striking member (10) is accelerated towards the transfer element (2), wherein a quick-action valve (16) releases the pneumatic medium under working pressure into the pressure channel (6),
**characterized in that** a control circuit (17) controls the opening period of the valve (16) as a function of at least one or a combination of the following parameters, namely the parameters of working pressure, striking frequency or pressure in the back-pressure chamber (8).

2. An instrument for the treatment of biological tissue, comprising a housing (4) in which are arranged a ballistic means for generating extracorporeal shock wave-like pressure waves and a transfer element (2) permanently placed on the biological tissue for coupling said pressure waves into the bodies of living beings, said transfer element (2) coupling non-focused, ballistically generated, shock wave-like pressure waves into said biological tissue, which are generated by a reciprocating striking member (10) accelerated to a final velocity of more than 5 m/s by means of a pneumatic medium under working pressure in a pressure channel (6) and striking said transfer element (2), the front part of said pressure channel (6) being connected with a back-pressure chamber (8) into which the pneumatic medium present distally of the striking member (10) can flow as the striking member (10) is accelerated towards the transfer element (2), wherein a quick-action valve (16) releases the pneumatic medium under working pressure into the pressure channel (6) in dependence on the striking frequency set,
**characterized in that** an opening (61) in the back-pressure chamber (8) limits the pressure building up in the back-pressure chamber (8).

3. The instrument of claim 2, **characterized in that** said opening (61) of the back-pressure chamber (8) is formed by a narrow orifice or by a pressure relief valve (60).

4. The instrument of one of claims 1 to 3, **characterized in that** the pressure in the back-pressure chamber (8) is controlled by a controllable pressure relief valve (60) as a function of one or a combination of the following parameters, i.e. the parameters of working pressure, striking frequency or pressure in the back-pressure chamber (8).

5. The instrument of claim 1 to 4, **characterized in that** said quick-action valve (16) releases said pneumatic medium from an intermediate storage (25) integrated in the housing (4), which storage buffers the medium at a predefined working pressure.

6. The instrument of one of claims 1 to 5, **characterized in that** said intermediate buffer (25) is arranged near the pressure channel (6) and is pneumatically connected to the pressure channel (6) via said valve (16) through a connecting conduit (19a, 19b) which is shorter than the length of the pressure channel (6) or short.

7. The instrument of one of claims 1 to 6, **characterized in that** the intermediate storage (25) is arranged in the supply conduit (48) for the pneumatic medium.

8. The instrument of one of claims 1 to 7, **characterized in that** a second valve releases a pneumatic pressure into the back-pressure chamber (8) for the return movement of the striking member (10).

9. The instrument of one of claims 1 to 8, **characterized in that** a single valve (16) controls both the forward and return movement of the striking member (10).

10. The instrument of one of claims 1 to 9, **characterized in that** the volume of the at least one intermediate storage (25) is at least half the volume of the pressure channel volume or at least half the volume of the pneumatic medium required in one stroke of the striking member (10).

11. The instrument of one of claims 1 to 10, **characterized in that** said valve (16) applies working pressure from the at least one intermediate storage (25) alternately to the pressure channel (6) or to the back-pressure chamber (8).

12. The instrument of one of claims 1 to 11, **characterized in that** said valve (16) alternately opens the pressure channel (6) or the back-pressure chamber (8) to atmosphere.

13. The instrument of one of claims 5 to 12, **characterized in that** the intermediate storage (25) comprises elastic walls or at least one elastic wall.

## Revendications

1. Instrument pour le traitement de tissus biologiques, comprenant un boîtier (4) dans lequel sont agencés un dispositif balistique servant à produire des ondes de pression extracorporelles, du type ondes de choc, et comprenant un élément de transmission (2) placé de façon permanente sur le tissu biologique et servant à l'injection des ondes de pression dans le corps d'êtres vivants, où l'élément de transmission (2) injecte dans le tissu biologique des ondes de pression, du type ondes de choc, non focalisées et produites balistiquement, ondes de pression qui peuvent être produites par un élément de percussion (10) qui est mobile par va-et-vient et accéléré dans un canal de pression (6) à une vitesse résiduelle élevée supérieure à 5 m/s par un milieu pneumatique se trouvant sous une pression de fonctionnement, et qui vient heurter l'élément de transmission (2), où la partie avant du canal de pression (6) est reliée à une chambre de contre-pression (8) dans laquelle le milieu pneumatique se trouvant en amont de l'élément de percussion (10), de façon distale, peut pénétrer lors de l'accélération de l'élément de percussion (10) venant heurter l'élément de transmission (2), où une soupape (16) à action rapide libère dans le canal de pression (6) le milieu pneumatique se trouvant sous la pression de fonctionnement,
**caractérisé en ce qu'**un circuit de commande (17) règle la durée d'ouverture de la soupape (16), en fonction d'au moins un paramètre ou d'une combinaison des paramètres suivants, à savoir les paramètres tels que la pression de fonctionnement ou la pression dans la chambre de contre-pression (8).

2. Instrument pour le traitement de tissus biologiques, comprenant un boîtier (4) dans lequel sont agencés un dispositif balistique servant à produire des ondes de pression extracorporelles, du type ondes de choc, et comprenant un élément de transmission (2) placé de façon permanente sur le tissu biologique et servant à l'injection des ondes de pression dans le corps d'êtres vivants, où l'élément de transmission (2) injecte dans le tissu biologique des ondes de pression, du type ondes de choc, non focalisées et produites balistiquement, ondes de pression qui peuvent être produites par un élément de percussion (10) qui est mobile par va-et-vient et accéléré dans un canal de pression (6) à une vitesse résiduelle élevée supérieure à 5 m/s par un milieu pneumatique se trouvant sous une pression de fonctionnement, et qui vient heurter l'élément de transmission (2), où la partie avant du canal de pression (6) est reliée à une chambre de contre-pression (8) dans laquelle le milieu pneumatique se trouvant en amont de l'élément de percussion (10), de façon distale, peut pénétrer lors de l'accélération de l'élément de percussion (10) venant heurter l'élément de transmission (2), où une soupape (16) à action rapide libère dans le canal de pression (6), en fonction de la fréquence de percussion réglée, le milieu pneumatique se trouvant sous la pression de fonctionnement,
**caractérisé en ce qu'**une ouverture (61) placée dans la chambre de contre-pression (8) limite la pression s'accumulant dans la chambre de contre-pression (8).

3. Instrument selon la revendication 2, **caractérisé en ce que** l'ouverture (61) en direction de la chambre de contre-pression (8) est formée par un diaphragme étroit ou par une soupape de surpression (60).

4. Instrument selon l'une quelconque des revendications 1 à 3, **caractérisé en ce que** la pression dans la chambre de contre-pression (8) est réglée par une soupape de surpression (60) réglable, en fonction d'au moins un paramètre ou d'une combinaison des paramètres suivants, à savoir les paramètres tels que la pression de fonctionnement, la fréquence de percussion et/ou la pression dans la chambre de contre-pression (8).

5. Instrument selon l'une quelconque des revendications 1 à 4, **caractérisé en ce que** la soupape (16) à action rapide libère le milieu pneumatique provenant d'un récipient de stockage temporaire (25) intégré au boîtier (4), lequel récipient stocke de façon temporaire le milieu, à une pression de fonctionnement prédéfinie.

6. Instrument selon l'une quelconque des revendications 1 à 5, **caractérisé en ce que** le récipient de stockage temporaire (25) est disposé à proximité du canal de pression (6) et est relié pneumatiquement au canal de pression (6), via la soupape (16), par une conduite de raccordement (19a, 19b) plus ou moins courte par rapport à la longueur du canal de pression (6).

7. Instrument selon l'une quelconque des revendications 1 à 6, **caractérisé en ce que** le récipient de stockage temporaire (25) est disposé dans la conduite d'alimentation (48) pour le milieu pneumatique.

8. Instrument selon l'une quelconque des revendications 1 à 7, **caractérisé en ce qu'**une deuxième soupape libère une pression pneumatique dans la chambre de contre-pression (8), pour le mouvement de retour de l'élément de percussion (10).

9. Instrument selon l'une quelconque des revendications 1 à 8, **caractérisé en ce qu'**une seule soupape (16) règle aussi bien le mouvement aller que le mouvement de retour de l'élément de percussion (10).

10. Instrument selon l'une quelconque des revendications 1 à 9, **caractérisé en ce que** le volume du récipient de stockage temporaire (25) au moins au nombre de un est égal au moins à la moitié du volume du canal de pression ou au moins à la moitié du volume de milieu pneumatique, nécessaire pour une course de l'élément de percussion (10).

11. Instrument selon l'une quelconque des revendications 1 à 10, **caractérisé en ce que** la soupape (16) alimente alternativement le canal de pression (6) ou la chambre de contre-pression (8), en pression de fonctionnement provenant du récipient de stockage temporaire (25) au moins au nombre de un.

12. Instrument selon l'une quelconque des revendications 1 à 11, **caractérisé en ce que** la soupape (16) ouvre vers l'atmosphère, alternativement le canal de pression (6) ou la chambre de contre-pression (8).

13. Instrument selon l'une quelconque des revendications 5 à 12, **caractérisé en ce que** le récipient de stockage temporaire (25) présente des parois élastiques ou au moins une paroi élastique.
